# EUROPEAN PATENT SPECIFICATION

(11) **EP 2 785 267 B1**
(45) Date of publication and mention of the grant of the patent: **12.01.2022**
(21) Application number: 11876682.3
(22) Date of filing: 21.12.2011
(51) Int. Cl.: A61B 90/50, B25J 9/00, A61B 34/30, B25J 18/00

(54) **APPARATUS AND METHOD FOR SUPPORTING A ROBOTIC ARM**
VORRICHTUNG UND VERFAHREN ZUR UNTERSTÜTZUNG EINES ROBOTERARMS
APPAREIL ET PROCÉDÉ DESTINÉS À SUPPORTER UN BRAS ROBOTIQUE

(30) Priority: 30.11.2011 US 201161565498 P; 14.12.2011 US 201161570560 P
(43) Date of publication of application: 08.10.2014
(73) Proprietor: Titan Medical Inc., Toronto, Ontario M5H 3M7 (CA)
(72) Inventor: SHVARTSBERG, Alexander, Oakville, Ontario L6M 4M7 (CA); CHARLES, Robert, A., New Boston, New Hampshire 03070 (US); MCCAFFREY, Robert, J., Hillsborough, New Hampshire 03244 (US); KENNEDY III, James, J., Deerfield, New Hampshire 03037 (US)
(74) Representative: Korenberg, Alexander Tal
(86) International application number: PCT/CA2011/001386
(87) International publication number: WO 2013/078529

(56) References cited:
- WO-A1-2004/070400
- WO-A1-2007/133065
- WO-A2-2010/068005
- WO-A2-2011/122862
- GB-A- 2 239 605
- GB-A- 2 239 605
- JP-A- 2003 079 638
- JP-A- 2008 017 903
- US-A- 5 749 362
- US-A- 5 749 362
- US-A1- 2007 156 122
- US-A1- 2008 019 607
- US-A1- 2008 213 077
- US-A1- 2009 041 565
- US-A1- 2011 077 523
- US-B1- 6 245 028
- US-B1- 6 245 028
- US-B1- 6 264 665
- US-B1- 6 264 665
- US-B1- 6 665 554

## Description

### FIELD

The present specification here relates in general to a field of robotic instruments, and more particularly, to a robotic system for use in surgery.

### BACKGROUND

With the gradual transition of medical surgery from the conventional process of making a long incision in the patient's body for performing a surgery to the next generation of surgery, i.e. minimal invasive surgery (MIS), continuous research is going on to develop and integrate robotic instruments in a system which can be used for MIS purposes. Such integration can help a surgeon perform a surgery in an error-free manner, and at the same time work in a realistic environment that gives the surgeon a feel of conventional surgery.

US5746362A describes a method and apparatus for determining positional information about an object and then using this information to position instruments in relation to the object.

US6264665B1 describes a system for ocular ultramicrosurgery which isolates the hands of a surgeon from the patient and provides increased positioning accuracy and speed to make practical the routine application of ultramicrosurgical operations to the eye.

US2009041565A1 describes a mechanism which constrains the spatial location of a working or a focal point of a tool and has a manipulator and a remote centre mechanism mounted thereon.

JP2008017903A describes an endoscope holding device composed of an arm to bring the distal end of the endoscope to the upper part of the affect region of a patient.

WO2010068005A2 describes a bed mount surgical robot for performing a surgical procedure on a patient lying on an operating bed using a surgical instrument mounted on an end portion of a robot arm.

JP2003079638A describes am operating robot comprising treatment instrument connected to the leading end of a manipulator

GB2239605A describes an article such as a surgical tool carried by a carriage which is movable along a curved track.

US2008019607A1 describes methods and systems to correct for various types of ring artifacts, including circular artifacts, partial ring artifacts, elliptical artifacts and variable intensity artifacts.

US2007156122A1 describes an apparatus including a linkage and a balancing mechanism coupled to the linkage around a pivotal joint.

WO2011122862A2 describes a fixed four-node link in which four links are joined together in a hinged fashion.

US2011077523 discloses another document relevant for the present invention

### SUMMARY

The present invention is defined by the appended independent claim 1. Preferred embodiments are disclosed in the dependent claims. In accordance with an aspect of the invention, there is provided an apparatus for medical procedures. The apparatus includes a base. The apparatus further includes a member having first and second ends. The first end connected to the base. The apparatus also includes a curved support configured to support a robotic arm. The curved support connected to the second end of the member.

The member may be configured to position the curved support relative to a surface of a surgical table.

The member may be articulable.

The curved support may be further configured to support the robotic arm at a plurality of locations.

The curved support may be configured to be positionable such that each location of the plurality of locations substantially equidistant from a target area.

The curved support is further configured to support a plurality of robotic arms.

The apparatus may further include a first robotic arm of the plurality of robotic arms interchangeable with a second robotic arm of the plurality of robotic arms.

The curved support may include a support rail disposed on the curved support.

The support rail may be connected to the second end of the member such that the curved support is configured to slide relative to the member.

The curved support includes a plurality of robotic arm rails disposed on the curved support.

The robotic arm rails are configured to slidably support the robotic arms.

The first end of the member may be pivotally connected to the base.

The member may include a first portion and a second portion. The first portion pivotally is connected to the second portion.

The curved support may be pivotally connected to the second end of the member.

The first end of the member may be rotatably connected to the base.

The curved support may be rotatably connected to the second end of the member.

In accordance with an aspect of the invention, there is provided a method according to appended claim 13.

### BRIEF DESCRIPTION OF THE DRAWINGS

Reference will now be made, by way of example only, to the accompanying drawings in which:
- Figure 1: is a perspective view of an operating theater according to an embodiment;
- Figure 2: is a perspective view of an operating theater according to another embodiment;
- Figure 3: is a view of a curved support positioned above a patient in accordance with the embodiment of Figure 2;
- Figure 4: is a perspective view of a curved support in accordance with another embodiment;
- Figure 5: is a cross sectional view of the curved support in accordance with the embodiment of Figure 4;
- Figure 6: is a perspective view of a curved support in accordance with another embodiment;
- Figure 7: is a cross sectional view of the curved support in accordance with the embodiment of Figure 6;
- Figure 8: is a perspective view of an operating theater according to an embodiment of the present invention;
- Figure 9: is a perspective view of a curved support in accordance with an embodiment of the present invention;
- Figure 10: is a cross sectional view of the curved support in accordance with the embodiment of Figure 9;
- Figure 11: is a view of a curved support and a plurality of robotic arms in accordance with another embodiment;
- Figure 12: is a view of a surgical apparatus in accordance with another embodiment; and
- Figure 13: is a flow chart of a method in accordance with an embodiment.

### DETAILED DESCRIPTION OF THE EMBODIMENTS

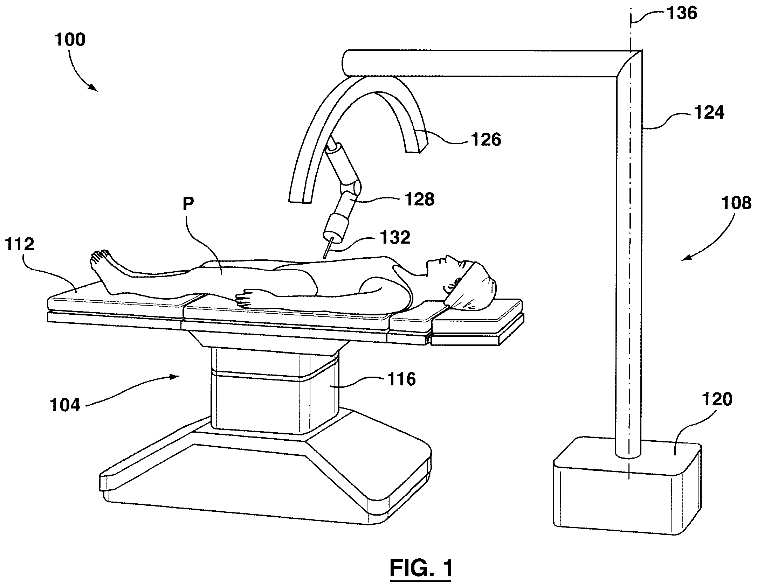

Referring to Figure 1, a schematic representation of an operating theater in a sterile environment for medical procedures such as Minimal Invasive Surgery (MIS) is shown at 100. It is to be understood that the operating theater 100 is purely exemplary and it will be apparent to those skilled in the art that a variety of operating theaters are contemplated. The operating theater 100 includes a surgical table 104 and a surgical apparatus 108. The surgical table 104 includes a surface 112 supported by a base 116. It is to be understood that the surgical table 104 is not particularly limited to any particular structural configuration. A patient P rests on the surface 112. The surgical apparatus 108 is for supporting a robotic arm 128, which in turn supports a robotic instrument 132. In the embodiment shown in Figure 1, the surgical apparatus 108 includes a base unit 120, a member 124, and a curved support 126.

In a present embodiment, the base unit 120 is generally configured to support other components of the surgical apparatus 108 which include the member 124, and the curved support 126. In addition, the base 120 also is configured to indirectly support the robotic arm 128 and the movements associated with the surgical apparatus 108 and connected components such as the robotic arm 128. In terms of providing physical support, the base unit 120 is mechanically structured to support the weight and movement of the member 124, and the curved support 126 in this embodiment. For example, the base unit 120 can be bolted to a fixed structure such as a wall, floor, or ceiling. Alternatively, the base unit 120 can have a mass and a geometry such that when the base unit 120 is free-standing, it will support the member 124, the curved support 126 and the robotic arm 128. In some embodiments, the base unit 120 can further include a moveable cart to provide easy movement of the surgical apparatus 108 around the operating theater 100.

In addition to providing structural support, the base unit 120 can also house various other components. For example, the base unit 120 can include mechanical controls (not shown), or electrical controls (not shown), or both. The mechanical controls can control gears, cables or other motion transfer mechanisms (not shown) connected to a motor, or other mechanical driver such as a hydraulic system, for moving various components of the surgical apparatus 108 and/or the robotic arm 128. In some embodiments, a control panel is disposed on the base 120 and configured to receive input associated with a movement of a component of the surgical apparatus 108, such as the member 124 or the robotic arm 128. In other embodiments, electrical signals or electromagnetic signals can be received from an external input device (not shown) to control the movements of other components of the surgical apparatus 108.

Referring again to Figure 1, the member 124 is generally configured to support the curved support 126, the robotic arm 128, and their associated movements. Therefore, in the present embodiment the member 124 acts as a support connected to the base 120 at a first end and to the curved support 126 at a second end. In terms of providing physical support, the member 124 is constructed of materials that are mechanically structured to support the weight of the curved support 126, the robotic arm 128, and their associated movements. For example, the member 124 can be constructed from materials such that it is rigid enough to be suspended above the patient P. Some examples of suitable materials from which the member 124 can be constructed include steel, titanium, aluminum, plastics, composites and other materials commonly used to provide structural support. In the present embodiment, the member 124 is configured such that it is positionable relative to the base unit 120. The member 124 includes a moveable joint at the base for providing a pivotal degree of freedom about an axis 136. It will now be understood that in embodiments where the member 124 is movable relative to the base, the movement of the member 124 can be controlled by the base unit 120 through various controls described above. In other embodiments, the member 124 can be rigidly fixed to the base 120 such that the member 124 can only be positioned by moving the base 120.

The curved support 126 is generally configured to support the robotic arm 128 and its associated movements. In the present embodiment, the curved support 126 is substantially "C-shaped" and is connected to the member 124 approximately at the center. It is to be understood that the connection point of the curved support 126 is not particularly limited. For example, the curved support 126 can be connected to the member 124 at one end of the curved support in certain applications. In terms of providing physical support, the curved support 126 can be constructed of materials that are mechanically structured to support the weight of the robotic arm 128 and its associated movements. Some examples of suitable materials from which the curved support 126 is constructed include steel, titanium, aluminum, plastics, composites and other materials commonly used to provide structural support. For example, the curved support 126 can be constructed from materials such that it is rigid enough to maintain its shape while being suspended above the patient P and connected to the member 124. In some embodiments, the curved support 126 can be configured such that it is positionable relative to the member 124. For example, the curved support 126 can include a plurality of mounts (not shown) disposed on the curved support 126 at which the curved support 126 can be connected to the member 124. It is to be appreciated that the plurality of mounts would provide a curved support 126 that is positionable relative to the member 124.

Referring again to Figure 1, in the present embodiment, the robotic arm 128 is generally configured to support the robotic instrument 132 and can include many configurations. As discussed above, the robotic arm 128 is mechanically structured to support and position the robotic instrument 132 both prior to and during surgery. Some examples of suitable materials from which the robotic arm 128 is constructed include steel, titanium, aluminum, plastics, composites and other materials commonly used to provide structural support. The robotic arm 128 is further configured such that the robotic instrument 132 is positionable relative to the base unit 120 and the surface 112. It is to be appreciated that the robotic arm 128 can move the robotic instrument away from the patient P prior to surgery such that the patient P can be properly positioned for the surgical procedure without interference from the robotic instrument 132. In addition, it is also to be appreciated that the robotic arm 128 can move the robotic instrument 132 during the surgical procedure to allow for the robotic instrument 132 to be positioned during surgery.

The degrees of freedom of the robotic arm 128 are not particularly limited and the robotic arm 128 can have any number of degrees of freedom as well as different types of degrees of freedom. A degree of freedom refers to an ability to move according to a specific motion. For example, a degree of freedom can include a rotation of the robotic arm 128 or a component thereof about a single axis. Therefore, for each axis of rotation, the robotic arm 128 is said to have a unique degree of freedom. Another example of a degree of freedom can include a translational movement along a path. For example, the robotic arm 128 can include an actuator for extending and contracting a portion of the robotic arm 128 linearly. It will now be apparent that each additional degree of freedom increases the versatility of the robotic arm 128. By providing more degrees of freedom, it will be possible to position the robotic arm 128 and the robotic instrument 132 in a wider variety of positions or locations to reach around obstacles. Furthermore, it is to be understood that in some embodiments, the member 124 can also include various degrees of freedom. It will now be apparent that each additional degree of freedom increases the versatility of the surgical apparatus 108.

The degrees of freedom of the robotic arm 128 fall generally into two different categories. One category includes non-surgical degrees of freedom. Non-surgical degrees of freedom refer to degrees of freedom which are adjusted prior to the surgical procedure. Once the surgical procedure has begun, the non-surgical degrees of freedom are generally not adjusted. Therefore, the purpose of the non-surgical degrees of freedom is to allow for the robotic instrument 132 to be positioned near a target area of patient P prior to surgery. The target area is the area where the surgical procedure is performed on the patient P. The other category of degrees of freedom includes surgical degrees of freedom. In contrast with non-surgical degrees of freedom, the surgical degrees of freedom are generally not adjusted prior to surgery and are intended to be adjusted during the surgical procedure to allow for the robotic instrument 132 to be moved accordingly as part of the surgical procedure. In general, surgical degrees of freedom are adjusted during surgery based on inputs received from an input device (not shown) under the control of a trained medical professional. For example, the base 120 can include a receiver for the inputs for controlling the surgical degrees of freedom. In some instances, it may be necessary to adjust the non-surgical degrees of freedom prior to surgery in order to configure the non-surgical degrees of freedom to a starting point prior to surgery.

In the present embodiment, the robotic instrument 132 is generally configured for performing MIS and is responsive to inputs received from an input device. In general, the input device is under the control of a trained medical professional performing the MIS. The configuration of the robotic instrument 132 is not particularly limited. For example, the robotic instrument 132 generally can move in accordance with at least one degree of freedom based on the received input. In addition, the robotic instrument can include working members which are also not particularly limited. It is to be appreciated that the number of degrees of freedom as well as the type and number of working members of the robotic instrument can be modified to meet the needs of the type of surgical procedure to be performed. For example, the robotic instrument 132 can include two working members wherein each working member corresponds to a jaw of a pair of forceps. In another example, the working members can be part of other surgical instruments such as scissors, blades, graspers, clip appliers, staplers, retractors, clamps or bi-polar cauterizers or combinations thereof. The robotic instrument 132 can also only include a single working member such as imaging equipment, for example a camera or light source, or surgical instruments such as scalpels, hooks, needles, catheters, spatulas or mono-polar cauterizers.

In general terms, the surgical apparatus 108 is configured to support the robotic arm 128 and robotic instrument 132 for performing MIS responsive to inputs from the input device (not shown). However, it is to be re-emphasized that the structure shown in Figure 1 is a schematic, non-limiting representation only. For example, although the surgical apparatus 108 shown in Figure 1 only supports one robotic arm 128, it is to be understood that the surgical apparatus 108 can be modified to support a plurality of robotic arms 128, each robotic arm of the plurality of robotic arms 128 having its own separate robotic instrument 132. Furthermore, it is also to be understood that where the surgical apparatus 108 supports a plurality of robotic arms 128, each of the robotic instruments 132 can have different structures. For example, the plurality of robotic instruments 132 can include a scalpel for cutting tissue and a pair of forceps for holding tissue. It is also to be understood that the surgical apparatus 108 may be part of a surgical system. In some embodiments, the surgical system may only include the surgical apparatus 108. Indeed, different configurations are contemplated herein.

In use, the robotic instrument 132 is positioned relative to the surface 112 on which the patient P rests by positioning the base 120 and then adjusting the member 124 and the robotic arm 128. In embodiments where the curved support 126 can also be positioned, the robotic arm 128 can be further positioned by positioning the curved support 126 relative to the member 124. It is to be understood that the mechanisms used to position the robotic instrument 132 are not particularly limited and that the structure shown in Figure 1 is merely a schematic, non-limiting representation. In the present embodiment, the member 124 can rotate about the axis 136. Therefore, the member 124 is rotatably connected to the base 120 such that the member 124 can be rotated about the axis 136 to position the curved support 126 above the patient P. In addition, the robotic arm 128 can be adjusted using the various non-surgical degrees of freedom to further position the robotic instrument 132 prior to surgery.

It is also to be appreciated that the ability to position the robotic instrument 132 by adjusting the robotic arm 128 and the member 124 is advantageous because it can facilitate positioning the patient P on the surgical table 104 prior to surgery without interference from the surgical apparatus 108. After the patient P is positioned, the surgical apparatus 108 is adjusted to allow the robotic instrument 132 to reach the target area. In particular, the target area refers to the general area where incisions are made and the robotic instruments are inserted into the patient P.

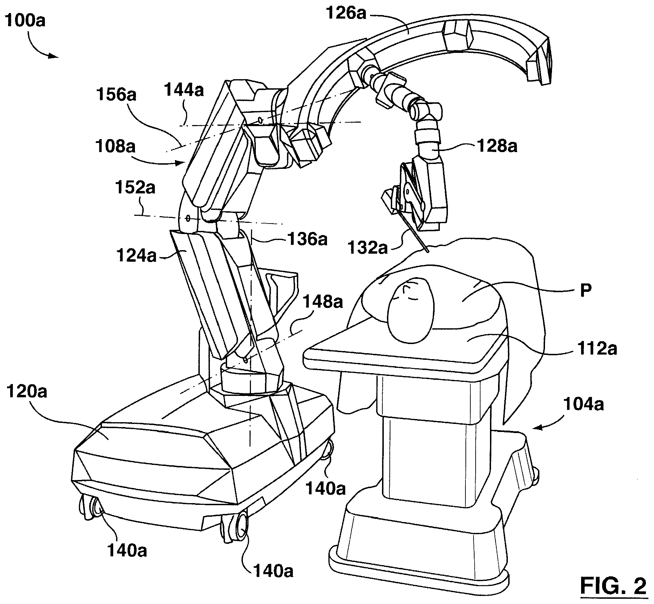

Referring to Figure 2, another embodiment of a surgical apparatus 108a is generally shown. Like components of the surgical apparatus 108a bear like reference to their counterparts in the surgical apparatus 108, except followed by the suffix "a". The surgical apparatus 108a includes a base unit 120a, a member 124a, and a curved support 126a for supporting a robotic arm 128a, which in turn supports a robotic instrument 132a.

In a present embodiment, the base unit 120a is generally configured to support other components of the surgical apparatus 108a which includes a member 124a, and a curved support 126a. In addition, the base 120a is also configured to support a robotic arm 128a connected to the curved support 126a. In terms of providing physical support, the base unit 120a is mechanically structured to support the weight and movement of the member 124a, the curved support 126a and the robotic arm 128a. In the present embodiment, the base unit 120a has a mass such that the base unit 120a can support the member 124a, the curved support 126a and the robotic arm 128a. Furthermore, in the embodiment shown in Figure 2, the base unit 120a includes a plurality of wheels 140a to provide easy movement of the entire surgical apparatus 108a around the operating theater 100a. In the present embodiment, each wheel 140a of the plurality of wheels preferably includes a locking mechanism (not shown) to hold the base stationary during the surgical procedure. In other embodiments, the based 120a can be modified such that a locking mechanism can only be included in only at least one wheel of the plurality of wheels 140a. In further embodiments, a separate locking mechanism such as a foot extending from the base can engage the floor to prevent movement of the base. Furthermore, it is also to be appreciated that in some embodiments, no locking mechanism may be required if the inertia of the base and relative frictional force associated with moving the surgical apparatus 108a is sufficient to prevent movement during a surgical procedure.

Referring again to Figure 2, the member 124a is generally configured to support the curved support 126a, the robotic arm 128a and their associated movements. In the present embodiment the member 124a is connected to the base 120a at a first end and to the curved support 126a at a second end. The member 124a of the present embodiment differs from the member 124 of the previous embodiment by including additional degrees of freedom. In the embodiment shown in Figure 2, the member 124a includes five degrees of freedom. The five degrees of freedom include two rotational degrees of freedom about a first rotation axis 136a and a second rotation axis 144a. In addition, the member 124a also includes three pivotal degrees of freedom where the member is articulated and pivotable about a first pivot axis 148a, second pivot axis 152a and third pivot axis 156a. It is to be understood that the five degrees of freedom provide a wide range of positions and orientations for the curved support 126a. For example, the curved support 126a can be raised and lowered by adjusting the member 124a about the pivot axes 148a, 152a, and 156a. In addition, the member 124a can also be independently pivoted about each pivot axis148a, 152a, and 156a. Therefore, the first pivot axis 148a can provide a pivotal connection between the member 124a and the base 120a. Similarly, the second pivot axis 152a can provide a pivotal connection between two portions of the member 124a. In addition, the third pivot axis 156a can provide a pivotal connection between the member 124a and the curved support 126a.

Furthermore, the orientation of the curved support 126a can be rotatably connected to the member 124a such that the curved support 126a can be adjusted using rotation about the rotation axis 144a. It is to be appreciated that rotation about the rotation axis 144a is advantageous for surgical procedures where the patient P is positioned on an inclined surface or where it is desired to configure the robotic arm 128a and the instrument 132a at a specific angle at the target area for a specific surgical procedure. It is to be understood that a wide range of further motions and positions of the curved support 126a can be obtained using various combinations of adjustments of the five degrees of freedom. Furthermore, the member 124a is capable of positioning the curved support away from the surgical table 104a to facilitate positioning the patient P. After the patient P is positioned on the surface 112a of the surgical table 104a, the member 124a can move the curved support 126a above the patient P and into position for the surgical procedure using the various independent degrees of freedom discussed above.

In terms of providing physical support, the member 124a is constructed of materials that are mechanically structured to support the weight of the curved support 126a, the robotic arm 128a and their associated movements. For example, the member 124a can be constructed from materials similar to those used for the member 124 of the previous embodiment. The five degrees of freedom associated with the member 124a in the present embodiment can be categorized as non-surgical degrees of freedom. As mentioned above, non-surgical degrees of freedom include degrees of freedom which are to be adjusted prior to the actual surgical procedure and fixed such that they are generally not adjusted during the surgical procedure. Therefore, since the member 124a includes various pivot and rotational degrees of freedom, locking mechanisms for each degree of freedom can be provided to prevent the member from moving during a surgical operation. The locking mechanisms are not particularly limited and can include a pin lock, a clamp, or a bolt. In other embodiments, the locking mechanism may be electromagnetically controlled. In some embodiments, the force of friction can be sufficient to hold the member in a given position.

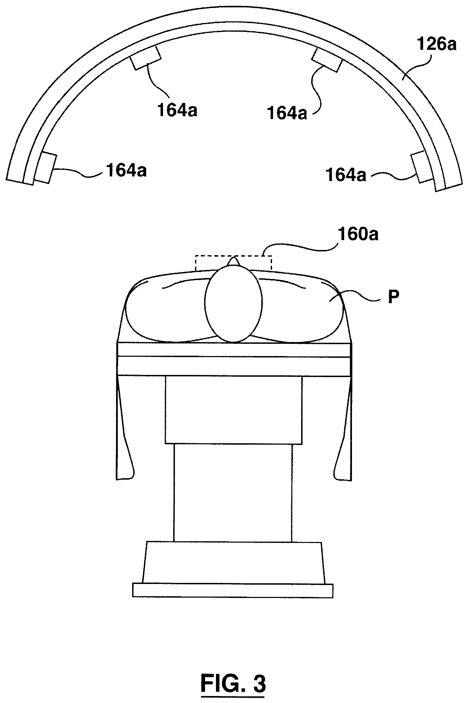

Referring to Figure 3, a schematic representation of the curved support 126a positioned above a patient P is generally shown in isolation from the remainder of theater 100a. The curved support 126a is generally configured to support the robotic arm 128a and its associated movements. In the present embodiment, the curved support 126a is connected to the member 124a approximately at one end (as shown in Figure 2). It is to be understood that that connection point of the curved support 126a to the member 124a is not particularly limited. Furthermore, the curved support 126a is generally configured to support the robotic arm 128a at a plurality of robotic arm mounts 164a along the curved support 126a. It is to be appreciated that the means for supporting the robotic arm 128a is not particularly limited and can include bolting the robotic arm to various positions, magnetically (or electromagnetically) attaching the robotic arm, or attaching the robotic arm using a pin locking mechanism. In other embodiments, the curved support 126a can be modified to be a curved robotic arm holder that uses a clamping system to hold the robotic arm 128a. As shown in Figure 3, in the present embodiment, the curved support 126a is generally positioned for a surgical procedure such that each robotic arm mount of the plurality of robotic arm mounts 164a is substantially equidistant from a target area 160a where incisions are made for the robotic instruments 132a to be inserted.

Referring again to Figure 2, in the present embodiment, the robotic arm 128a is generally configured to support the robotic instrument 132a. Both the robotic arm 128a and the robotic instrument 132a are substantially similar to the robotic arm 128 and the robotic instrument 132 of the previous embodiment. The degrees of freedom of the robotic arm 128a are not particularly limited and the robotic arm 128a can have any number of degrees of freedom as well as different types of degrees of freedom as discussed above in connection with the previous embodiment.

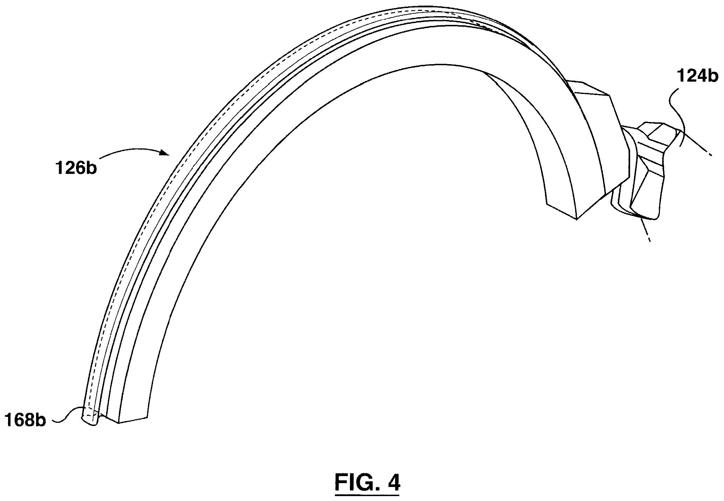

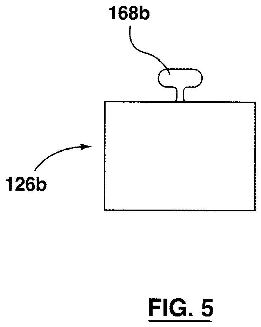

Referring to Figures 4 and 5, another embodiment of a curved structure 126b is shown. Like components of the curved structure 126b bear like reference to their counterparts in the curved structure 126a, except followed by the suffix "b". The curved support 126b is generally configured to support a robotic arm (not shown in Figure 4) and its associated movements.

In the present embodiment the curved support 126b includes a support rail 168b which is configured to be slidably connected to a member 124b. It is to be understood that the support rail 168b is configured to allow the curved support 126b to slide relative to the member 124b. Therefore, an additional non-surgical degree of freedom will be provided to allow for the robotic instrument (not shown) to be positioned near a target area. Since the support rail 168b provides a non-surgical degree of freedom which should not be permitted to move during a surgical procedure, a locking mechanism is also generally included to prevent movement. It is to be appreciated that the configuration of the support rail 168b is not particularly limited. In the present embodiment shown in Figures 4 and 5, the support rail 168b extends substantially along the entire length of the curved support 126b. In other embodiments, the support rail 168b can only extend for a portion of the length of the curved support 126b. Alternatively, the support rail 168b can also extend beyond the length of the curved support 126b in some embodiments to provide a larger range of motion. In other embodiments still, the curved support 126b can be modified to use another mechanism to provide a slidable motion. For example, other mechanisms can include the use of slots or tracks which allow for a sliding motion.

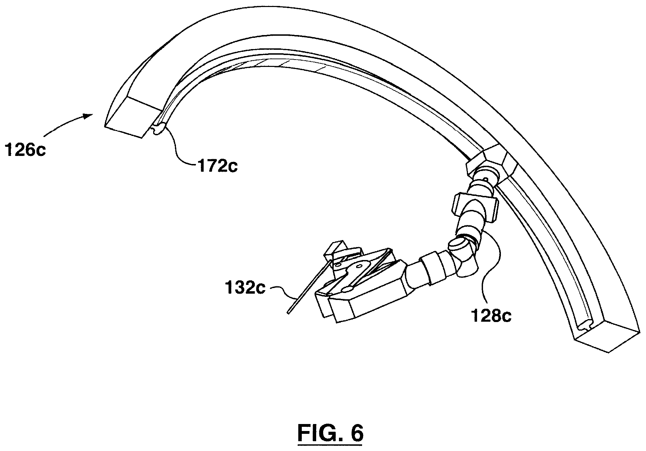

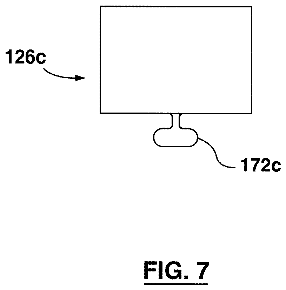

Referring to Figures 6 and 7, another embodiment of a curved structure 126c is shown. Like components of the curved structure 126c bear like reference to their counterparts in the curved structure 126a, except followed by the suffix "c". The curved support 126c is generally configured to support a robotic arm and its associated movements.

In the present embodiment the curved support 126c includes a robotic arm rail 172c which is configured to support a robotic arm 128c slidably connected to the curved support 126c. It is to be understood that the robotic arm rail 172c is configured to allow the robotic arm 128c to slide relative to the curved support 126c. Therefore, an additional non-surgical degree of freedom will be provided to allow for a robotic instrument 132c to be positioned near a target area. Since the robotic arm rail 172c provides a non-surgical degree of freedom, a locking mechanism is also generally included to prevent movement during the surgical procedure. It is to be appreciated that the configuration of the robotic arm rail 172c is not particularly limited. In the present embodiment shown in Figures 6 and 7, the robotic arm rail 172c extends substantially along the entire length of the curved support 126c. In other embodiments, the robotic arm rail 172c can only extend for a portion of the length of the curved support 126c. Alternatively, the robotic arm rail 172c can also extend beyond the length of the curved support 126c in some embodiments to provide a larger range of motion. In other embodiments still, the curved support 126c can be modified to use another mechanism to provide a slidable motion.

For example, other mechanisms can include the use of slots or tracks which allow for a sliding motion.

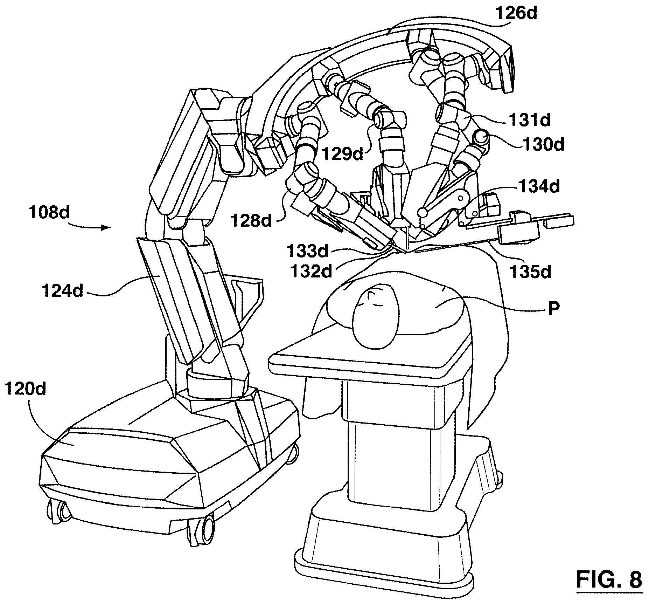

Referring to Figure 8, an embodiment of the present invention of a surgical apparatus 108d is generally shown. Like components of the surgical apparatus 108d bear like reference to their counterparts in the surgical apparatus 108a, except followed by the suffix "d". The surgical apparatus 108d includes a base unit 120d, a member 124d, and a curved support 126d for supporting a plurality of robotic arms 128d, 129d, 130d and 131d. The robotic arms 128d, 129d, 130d and 131d further support a plurality of robotic instruments 132d, 133d, 134d, and 135d, respectively. It is to be understood the robotic instruments 132d, 133d, 134d, and 135d generally have different structures which include different types of surgical instruments. Therefore, it is to be appreciated that the plurality of arms allows for different tools to be used in a surgical procedure.

In the present embodiment, it is to be understood that the robotic arms 128d, 129d, 130d and 131d can be interchanged with each other. Therefore, for surgical procedures which contemplate placement of the robotic arms 128d, 129d, 130d and 131d in different positions, the change can be made prior to the surgical procedure. Furthermore, it is to be appreciated that when the curved support 126d is designed such that each robotic arm mount of the curved support 126d is substantially equidistant from a target area, the interchanging of robotic arms 128d, 129d, 130d and 131d is facilitated since the length of each of the robotic arms 128d, 129d, 130d and 131d would be similar.

It is also to be appreciated that the design of the curved support 126d allows for the lengths of the robotic arms 128d, 129d, 130d and 131d to be decreased when compared with using a straight robotic arm support. Therefore, the physical footprint and volume of space occupied by the surgical apparatus will be decreased since the robotic arms would have to extend further to reach the target area. It is to be understood that this is particularly advantageous in an operating theater where space is often limited due to the large amount of equipment used in a surgical procedure.

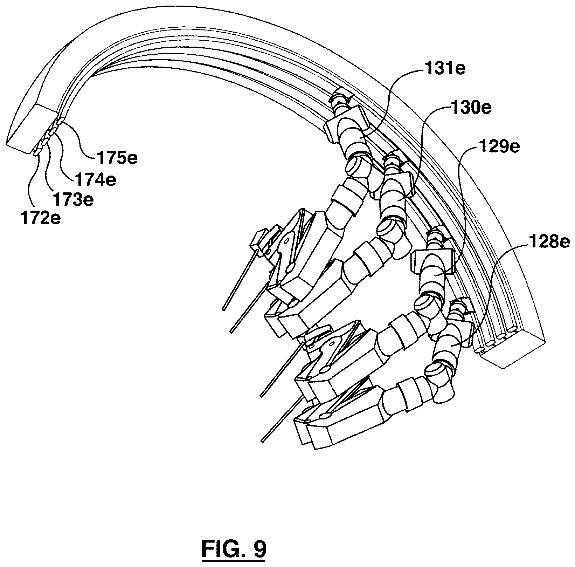

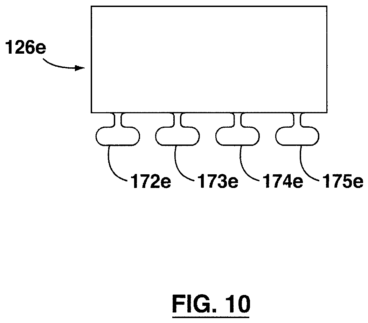

Referring to Figures 9 and 10, an embodiment of the present invention of a curved structure 126e is shown. Like components of the curved structure 126e bear like reference to their counterparts in the curved structure 126c, except followed by the suffix "e". The curved support 126e is generally configured to support a plurality of robotic arms 128e, 129e, 130e and 131e and their associated movements.

In the present embodiment the curved support 126e includes a plurality of robotic arm rails 172e, 173e, 174e, and 175e which are slidably connected to the robotic arms 128e, 129e, 130e and 131e, respectively. It is to be understood that the robotic arm rails 172e, 173e, 174e, and 175e are configured to allow the robotic arms 128e, 129e, 130e and 131e, respectively, to slide independently relative to the curved support 126e. Therefore, an additional non-surgical degree of freedom will be provided for each robotic arm. Therefore, since the robotic arm arms 128e, 129e, 130e and 131e provide a non-surgical degree of freedom, locking mechanisms are also generally included to prevent movement during the surgical procedure. Furthermore, it is to be appreciated that since each of the robotic arms 128e, 129e, 130e and 131e is connected to a separate track, the robotic arms 128e, 129e, 130e and 131e interchange positions by simply sliding past each other if space permits.

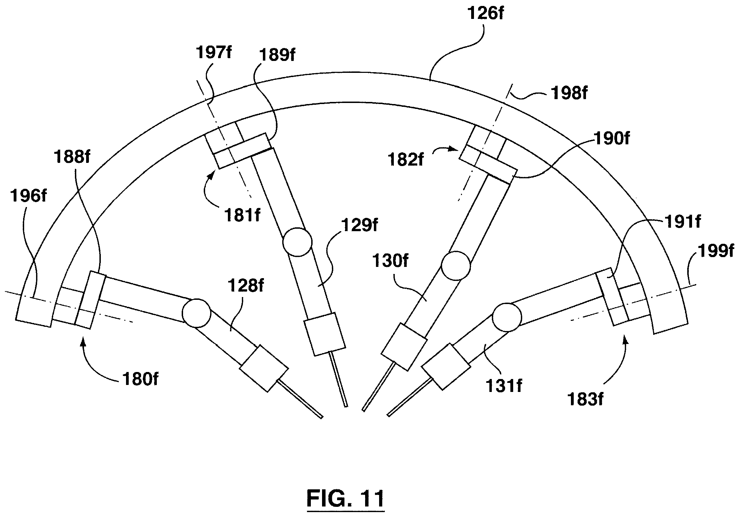

Referring to Figure 11, another embodiment of a plurality of robotic arms 128f, 129f, 130f and 131f is shown. Like components bear like reference to their counterparts, except followed by the suffix "f". The plurality of robotic arms 128f, 129f, 130f and 131f are generally configured allow for an addition non-surgical degree of freedom using off-axis apparatus 180f, 181f, 182f, and 183f.

In the present embodiment, the off-axis apparatus 180f, 181f, 182f, and 183f provides extension members 188f, 189f, 190f, and 191f, respectively, which rotate about axes 196f, 197f, 198f, and 199f. It is to be understood that the rotation about the axes 196f, 197f, 198f, and 199f allows the robotic arms 128f, 129f, 130f and 131f to be staggered relative to the curved support 126f. Therefore, it is to be appreciated that the robotic arms 128f, 129f, 130f and 131f can be positioned closer to each other for applications which require robotic instruments (not shown) to be in closer proximity such as oral surgery applications thus providing for additional non-surgical degrees of freedom.

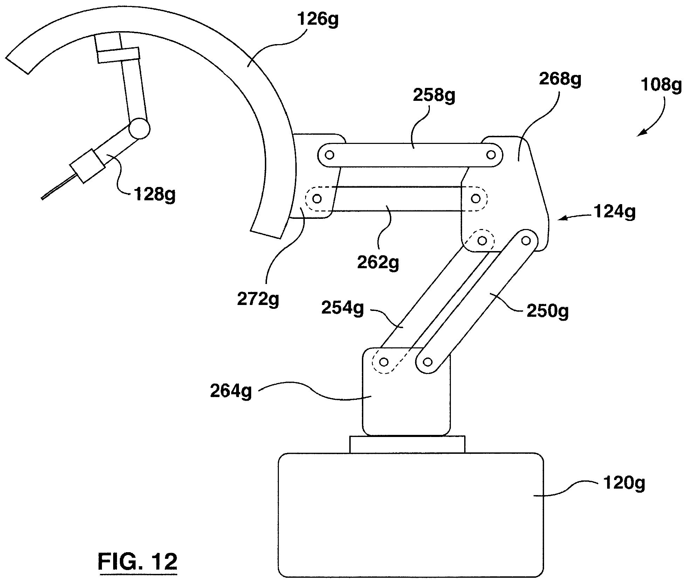

Referring to Figure 12, yet another embodiment of a surgical apparatus 108g is generally shown. The surgical apparatus 108g includes a base unit 120g, a member 124g, and a curved support 126g for supporting a robotic arm 128g.

In the present embodiment, the member 124g is generally configured to support the curved support 126g, the robotic arm 128g and their associated movements. The member 124g is connected to the base 120g at a first end and to the curved support 126g at a second end. The member 124g of the present embodiment differs from the member 124a of a previous embodiment by including four-bar linkages. In the present embodiment, a first bar 250g and a second bar 254g are pivotally connected to a first connector 264g and a second connector 268g of the member 124g to form a first four-bar linkage. In addition, a third bar 258g and a fourth bar 262g are pivotally connected to the second connector 268g and a third connector 272g of the member 124g to form a second four-bar linkage as shown in Figure 12. It is to be understood that the four-bar linkage system shown in Figure 12 allows for the orientation of the curved support 126g to remain substantially constant as the position of the curved support 126g is adjusted.

It is to be understood that combinations and subsets of the embodiments and teachings herein are contemplated. As a non-limiting example, the curved support 126d of the surgical apparatus 108d can be modified with teachings of the curved support 126c having a single robotic arm rail 172c. It is to be appreciated that in this embodiment, the robotic arms 128d, 129d, 130d and 131d would no longer be able to interchange positions by sliding past each other since the robotic arms 128d, 129d, 130d and 131d would then share the same track.

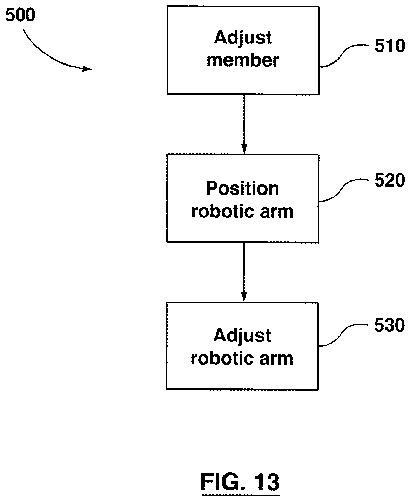

In another variation of the surgical apparatus 108d, all non-surgical degrees of freedom can be adjusted using a plurality of motors (not shown). For example, each motor can adjust a non-surgical degree of freedom based on input from an input device. Alternatively, each motor can also be used to provide assistance for adjusting a non-surgical degree of freedom based on input from a force feedback system. It is to be understood that a combination of the two types of motor assistance is also contemplated. Furthermore, in some embodiments, a control console (not shown) can store various pre-configured positions for a specific patient or a specific procedure. The pre-configured positions can involve specific positions of the non-surgical degrees of freedom specific to either a patient or a particular type of surgery. Therefore, the non-surgical positioning of the robotic arms 128d, 129d, 130d and 131d as well as the member 124d and curved support 126d can be calculated and stored using a simulation program prior to a surgical procedure. For example, the simulation program can use patient specific data such as Magnetic Resonance Imaging (MRI), CT Scan and/or X-ray results to calculate a pre-configured position. It is to be appreciated that by using pre-configured positions determined outside of an operating theater, valuable time spent in the operating theater can be saved. Referring now to Figure 13, a method for positioning a robotic instrument for performing robotic surgery is shown generally at 500. Method 500 can perform on one of the surgical apparatus described above as well as any variations contemplated. For the purposes of this discussion, the method 500 will be discussed primarily in connection with the surgical apparatus 108 shown in Figure 1. It is to be emphasized that the reference to the surgical apparatus 108 does not limit the application of the method 500 discussed below to only the surgical apparatus 108. Furthermore, the method 500 can be carried out using a processor programmed to control motors for adjusting non-surgical degrees of freedom.

Block 510 comprises adjusting the member 124 to position the curved support 126 above the patient P. The manner in which the adjustment is carried out is not particularly limited. In the present example, the member can only be rotated about the axis 136. It is to be understood that in other embodiments, the member can have more degrees of freedom to allow for further adjustments. In other embodiments still, a motor can be used to facilitate the adjustment.

Block 520 comprises positioning the robotic arm 128 at a location on the curved support 126. As discussed above, the robotic arm 128 can be positioned either by connecting the robotic arm to the desired location. For example, discrete robotic arm mounts can be provided as in the curved support 126a. In other embodiments such as the one including the curved support 126c, positioning the robotic arm 128c can involve sliding the robotic arm 128c along a robotic arm rail 172c. It is to be understood that in another variation, the robotic arm 128c can be modified to interact with a leadscrew driven by a motor to provide motion along the robotic arm rail 172c.

Block 530 comprises adjusting the robotic arm 128 in accordance with non-surgical adjustments such that the robotic instrument 132 is within range of a target area. The manner in which the adjustment is carried out is not important. In the present example, the robotic arm 128 includes joints which can be adjusted according to a non-surgical degree of freedom and locked in place. In other examples, motors can drive a gear, lead screw or harmonic drive to carry out the adjustment.

It is to be understood that variations of the method 500 are contemplated. As a non-limiting example, the method can additionally involve adjusting the curved support 126c relative to the member. In one embodiment, the curved support 126c can include a support rail configured to slidably connect to the member 124. As another non-limiting example, the method can also involve storing pre-determined position to reduce the amount of time needed in the operating theater.

While specific embodiments have been described and illustrated, such embodiments should be considered illustrative only and should not serve to limit the accompanying claims.

## Claims

1. An apparatus (108) for medical procedures, the apparatus comprising:
a plurality of robotic arms (128e, 129e, 130e, 131e);
a base (120);
a member (124) having first and second ends, the first end connected to the base; and
a curved support (126) connected to the second end of the member, wherein the curved support comprises a plurality of robotic arm rails (172e, 173e, 174e, 175e) each robotic arm rail configured to slidably support a respective one of the plurality of robotic arms such that the robotic arms can interchange positions by sliding past each other.

2. The apparatus of claim 1, wherein the member is configured to position the curved support.

3. The apparatus of claim 2, wherein the member is articulable.

4. The apparatus of claim 1, wherein each curved rail is configured to support the respective robotic arm at a plurality of locations.

5. The apparatus of claim 4, wherein the curved support is configured to be positionable such that each location of the plurality of locations substantially equidistant from a target area.

6. The apparatus of claim 1, wherein a first robotic arm of the plurality of robotic arms is interchangeable with a second robotic arm of the plurality of robotic arms.

7. The apparatus of claim 1, wherein the curved support comprises a support rail (168b) disposed on the curved support and the support rail is connected to the second end of the member such that the curved support is configured to slide relative to the member.

8. The apparatus of claim 1, wherein the first end of the member is pivotally connected to the base.

9. The apparatus of claim 1, wherein the member comprises a first portion and a second portion, the first portion pivotally connected to the second portion.

10. The apparatus of claim 1, wherein the curved support is pivotally connected to the second end of the member.

11. The apparatus of claim 1, wherein the first end of the member is rotatably connected to the base.

12. The apparatus of claim 1, wherein the curved support is rotatably connected to the second end of the member.

13. A method for positioning a robotic instrument for performing robotic surgery, prior to a surgical procedure, the method comprising:
adjusting a member (124) to position a curved support (126) comprising a plurality of robotic arm rails (172e, 173e, 174e, 175e) each configured to slidably support a respective robotic arm (128e, 129e, 130e, 131e) such that the robotic arms can interchange positions by sliding past each other;
positioning a robotic arm at a location along a respective robotic arm rail on the curved support; and
adjusting the robotic arm in accordance with a non-surgical adjustment such that the robotic instrument is within range of a target area.

## Patentansprüche

1. Vorrichtung (108) für medizinische Vorgänge, wobei die Vorrichtung Folgendes umfasst:
eine Vielzahl von Roboterarmen (128e, 129e, 130e, 131e),
eine Basis (120),
ein Glied (124) mit einem ersten und einem zweiten Ende, wobei das erste Ende mit der Basis verbunden ist, und
eine gekrümmte Stütze (126), die mit dem zweiten Ende des Glieds verbunden ist, wobei die gekrümmte Stütze eine Vielzahl von Roboterarmschienen (172e, 173e, 174e, 175e) umfasst, wobei jede Roboterarmschiene dazu ausgestaltet ist, einen jeweiligen der Vielzahl von Roboterarmen verschiebbar zu stützen, so dass die Roboterarme Positionen tauschen können, indem sie aneinander vorbeigleiten.

2. Vorrichtung nach Anspruch 1, wobei das Glied zur Positionierung der gekrümmten Stütze ausgestaltet ist.

3. Vorrichtung nach Anspruch 2, wobei das Glied gelenkig bewegbar ist.

4. Vorrichtung nach Anspruch 1, wobei jede gekrümmte Schiene dazu ausgestaltet ist, den jeweiligen Roboterarm an einer Vielzahl von Orten zu stützen.

5. Vorrichtung nach Anspruch 4, wobei die gekrümmte Stütze dazu ausgestaltet ist, so positionierbar zu sein, dass jeder Ort der Vielzahl von Orten im Wesentlichen den gleichen Abstand von einem Zielbereich hat.

6. Vorrichtung nach Anspruch 1, wobei ein erster Roboterarm der Vielzahl von Roboterarmen mit einem zweiten Roboterarm der Vielzahl von Roboterarmen austauschbar ist.

7. Vorrichtung nach Anspruch 1, wobei die gekrümmte Stütze eine Stützschiene (168b) umfasst, die an der gekrümmten Stütze angeordnet ist, wobei die Stützschiene mit dem zweiten Ende des Glieds verbunden ist, so dass die gekrümmte Stütze zum Gleiten bezüglich des Glieds ausgestaltet ist.

8. Vorrichtung nach Anspruch 1, wobei das erste Ende des Glieds schwenkbar mit der Basis verbunden ist.

9. Vorrichtung nach Anspruch 1, wobei das Glied einen ersten Abschnitt und einen zweiten Abschnitt umfasst, wobei der erste Abschnitt schwenkbar mit dem zweiten Abschnitt verbunden ist.

10. Vorrichtung nach Anspruch 1, wobei die gekrümmte Stütze schwenkbar mit dem zweiten Ende des Glieds verbunden ist.

11. Vorrichtung nach Anspruch 1, wobei das erste Ende des Glieds drehbar mit der Basis verbunden ist.

12. Vorrichtung nach Anspruch 1, wobei die gekrümmte Stütze drehbar mit dem zweiten Ende des Glieds verbunden ist.

13. Verfahren zur Positionierung eines Roboterinstruments zur Durchführung von Roboterchirurgie vor einem chirurgischen Vorgang, wobei das Verfahren Folgendes umfasst:
Verstellen eines Glieds (124) zur Positionierung einer gekrümmten Stütze (126), die eine Vielzahl von Roboterarmschienen (172e, 173e, 174e, 175e) umfasst, die jeweils dazu ausgestaltet sind, einen jeweiligen Roboterarm (128e, 129e, 130e, 131e) verschiebbar zu stützen, so dass die Roboterarme Positionen tauschen können, indem sie aneinander vorbeigleiten,
Positionieren eines Roboterarms an einem Ort entlang einer jeweiligen Roboterarmschiene an der gekrümmten Stütze und
Verstellen des Roboterarms in Übereinstimmung mit einem nicht chirurgischen Verstellen, so dass das Roboterinstrument in Reichweite eines Zielbereichs ist.

## Revendications

1. Appareil (108) pour des procédures médicales, l'appareil comprenant :
une pluralité de bras robotiques (128e, 129e, 130e, 131e) ;
une base (120) ;
un élément (124) ayant des première et seconde extrémités, la première extrémité étant reliée à la base ; et
un support incurvé (126) relié à la seconde extrémité de l'élément, le support incurvé comprenant une pluralité de rails de bras robotiques (172e, 173e, 174e, 175e), chaque rail de bras robotique étant configuré pour supporter de manière coulissante un bras respectif de la pluralité de bras robotiques de telle sorte que les bras robotiques peuvent changer de position en glissant les uns devant les autres.

2. Appareil selon la revendication 1, l'élément étant configuré pour positionner le support incurvé.

3. Appareil selon la revendication 2, l'élément étant articulable.

4. Appareil selon la revendication 1, chaque rail incurvé étant configuré pour supporter le bras robotique respectif à une pluralité d'emplacements.

5. Appareil selon la revendication 4, le support incurvé étant configuré pour être positionnable de telle sorte que chaque emplacement de la pluralité d'emplacements est sensiblement équidistant d'une zone cible.

6. Appareil selon la revendication 1, un premier bras robotique de la pluralité de bras robotiques étant interchangeable avec un second bras robotique de la pluralité de bras robotiques.

7. Appareil selon la revendication 1, le support incurvé comprenant un rail de support (168b) disposé sur le support incurvé et le rail de support étant relié à la seconde extrémité de l'élément de telle sorte que le support incurvé est configuré pour glisser par rapport à l'élément.

8. Appareil selon la revendication 1, la première extrémité de l'élément étant reliée de manière pivotante à la base.

9. Appareil selon la revendication 1, l'élément comprenant une première partie et une seconde partie, la première partie étant reliée de manière pivotante à la seconde partie.

10. Appareil selon la revendication 1, le support incurvé étant relié de manière pivotante à la seconde extrémité de l'élément.

11. Appareil selon la revendication 1, la première extrémité de l'élément étant reliée de manière rotative à la base.

12. Appareil selon la revendication 1, le support incurvé étant relié de manière rotative à la seconde extrémité de l'élément.

13. Procédé de positionnement d'un instrument robotique pour réaliser une chirurgie robotique, avant une procédure chirurgicale, le procédé comprenant :
l'ajustement d'un élément (124) pour positionner un support incurvé (126) comprenant une pluralité de rails de bras robotiques (172e, 173e, 174e, 175e) configurés chacun pour supporter de manière coulissante un bras robotique respectif (128e, 129e, 130e, 131e) de telle sorte que les bras robotiques peuvent changer de position en glissant les uns devant les autres ;
le positionnement d'un bras robotique à un emplacement le long d'un rail de bras robotique respectif sur le support incurvé ; et
l'ajustement du bras robotique en fonction d'un réglage non chirurgical de telle sorte que l'instrument robotique se situe dans le champ d'une région cible.
